# EUROPEAN PATENT APPLICATION

(11) **EP 2 368 498 A1**
(43) Date of publication of application: **28.09.2011**
(21) Application number: 10158059.5
(22) Date of filing: 26.03.2010
(51) Int. Cl.: A61B 6/14, A61C 13/00

(54) **Method for deriving shape information of a person's dentition**

(71) Applicant: Stichting voor de Technische Wetenschappen, 3527 JP Utrecht (NL); Stichting Katholieke Universiteit, Radboud University Nijmegen Medical Centre, 6500 HB Nijmegen (NL)
(72) Inventor: Bergé, Stefaan Jozef, 47800 Krefeld (DE); Kuijpers-Jagtman, Anne-Marie, 6581 NA Malden (NL); Rangel, Frits, 6525 JJ Nijmegen (NL); Maal, Thomas, 6511 AK Nijmegen (NL)
(74) Representative: BiiP cvba

(57) **Abstract**

The present invention is related to a method for deriving shape information of a person's dentition. The method comprises the steps of
- obtaining a first data set by taking a 3D scan of the person's dentition with markers fixed in the person's intra-oral cavity,
- digitizing an impression of the person's dentition with the markers fixed, yielding a second data set,
- fusing the first and the second data set based on corresponding pairs of the markers,
- deriving the shape information from the fused first and second data sets.

## Description

### Field of the Invention

The present invention generally relates to methods to visualize the anatomy of the head with detailed and accurate visualization of the shape of teeth and surrounding soft tissue.

### Background of the Invention

With volumetric imaging techniques, such as (multi-slice and cone-beam) CT, MRI, etc, it is possible to obtain an accurate three-dimensional (3D) representation of the patient's head in three dimensions. Using 3D clinical software, diagnosis can be done, and treatments, including surgical treatments, can be planned digitally and can be transferred to the patient, optically through e.g. navigation or with patient specific tooling, which reduces errors and optimizes the efficiency and efficacy in relation to materials, construction of appliances, and hand-skills.

For dental applications, a clear visualization of the patient's dentition and soft tissues, also the intra-oral soft tissues, is important. However, when scanning the patient with the volumetric imaging techniques does not give enough information to accurately describe and visualize the teeth surfaces and the soft tissues.

Since the introduction of 3D imaging devices, several procedures have been developed to integrate a digital dental cast into 3D computerized models.

WO03/028577 describes a method to generate surgical splints for orthognathic surgery. As part of this method, a dental cast is fused with the patient scan, using a bite jig, with fiducial markers attached to it. In this bite jig the occlusion was recorded in centric relation. The patient wore the bite jig when the CBCT scan was made and afterwards the bite jig was scanned together with the impressions. Both data sets were matched, using the fiducial markers as reference points. The disadvantage of this method is that the fiducial markers are positioned outside the mouth and are giving a distortion of the soft tissues. In this way a reliable judgment of the soft tissues of the patient at rest cannot be obtained.

International application W02008/128720 developed a triple scan method, using an impression tray in which both the upper and lower jaw are registered. For this procedure, a volumetric scan was made of the patient in rest, using a wax bite to control the occlusion. Next, a second scan was made of the patient, with the impression tray placed in the mouth. Finally the impression tray was scanned separately. Those scans are then combined to have detailed teeth shape information fused in the patient scan. The major disadvantage of this method is that: (1) two CBCT scans need to be made of the patient, which gives unnecessary x-ray exposure and (2) special care needs to be taken to check whether the patient bites down the same in the wax bite and the impression tray.

Consequently, there is a need to overcome the shortcomings of the prior art methods.

### Aims of the invention

The present invention aims to provide a method to create a skull model with a detailed visualization of the dentition and surrounding soft tissue without deforming the facial soft tissues and whereby the x-ray exposure of the patient is limited.

### Summary

The present invention relates to a method for deriving shape information of a person's dentition. The method comprises the steps of
- obtaining a first data set from a three-dimensional scan of the person's dentition with markers fixed in the person's intra-oral cavity,
- digitizing an impression of the person's dentition with the markers fixed, yielding a second data set,
- fusing the first and the second data set based on corresponding pairs of markers,
- deriving the shape information from the fused first and second data sets.

In a first method step a first data set is derived from a volumetric scan of at least the person's dentition, but it also can be a scan of the person's head. The 3D scan can be made available in a preliminary step, wherein first the markers are fixed to the patient's intra-oral cavity. The scan is taken while the person's head holds the markers. In one embodiment this preliminary step is comprised in the method of the invention. In another preliminary step after acquiring the 3D scan an impression of the patient's dentition is made during which the markers are transferred from the patient towards the impression. Also this step of actually making the impression after acquiring the volumetric scan is in one embodiment comprised in the method of this invention. The impression of the person's dentition with the markers fixed is digitized, resulting in a second data set. In a next step the first and the second data set are then fused based on corresponding pairs of markers in the two data sets. Then the shape information is derived.

In a preferred embodiment of the invention the markers are fixed to soft tissue in the intra-oral cavity, e.g. the gingiva. Alternatively, the markers are fixed to the teeth or a restoration of the teeth.

The step of digitizing the impression is advantageously performed with a volumetric imaging technique or with a surface scanning technique.

The markers are preferably made of titanium.

In an advantageous embodiment at least three markers are used. The at least three markers are preferably placed in a random pattern.

In another preferred embodiment the method comprises a step of adding colour information to the impression.

In one embodiment the method further comprises a step of extracting the dentition from the digitized impression. A further step is then typically performed wherein the extracted dentition replaces the dentition in the first data set.

The present invention also relates to a program, executable on a programmable device containing instructions, which when executed, perform the method as previously described.

### Brief Description of the Drawings

Fig. 1 represents a flow chart of an embodiment of the method according to the present invention.

Fig. 2 represents an intra-oral view of a patient with markers placed on the gingiva.

Fig. 3 illustrates a surface representation of the patient's skull out of CT imaging. Image B highlights the detected markers.

Fig. 4 represents a surface representation of the impression out of a volumetric image dataset acquired with CT. Image B highlights the position of the markers.

Fig. 5 represents an overview of a possible implementation of the workflow.

Fig. 6 represents the optional step of replacing the teeth shape of the patient scan by the teeth shape of the digitized impression.

### Detailed Description of the Invention

The present invention describes a method to solve the above-mentioned problems of the prior art with only one patient CT-scan and without deforming the facial soft tissues.

Dental impressions can be digitized with various techniques, such as volumetric imaging techniques as well as surface scanning techniques, resulting in a digital representation of the teeth and surrounding soft tissues which are part of the impression. This digitized representation of the dental impression needs to be fused with the volumetric patient scan. By doing so, accurate information is obtained about the teeth and surrounding soft tissues into the patient scan.

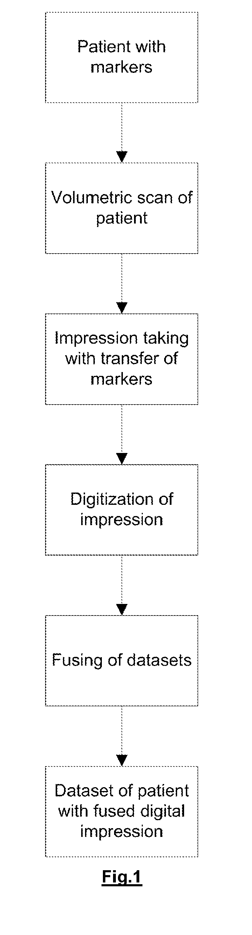

An overview of the proposed workflow is depicted in Fig.1. In the method markers are fixed in the intra-oral cavity. One option is to place them on soft tissue like the gingiva. Alternatively the markers can be fixed to the teeth or the restoration of the teeth if present. These markers are detectable in the volumetric patient scan. Then the patient is scanned. In a next step, an impression of the patient's dentition is made and the markers now engage with the impression material instead of the gingiva. The impression is digitized and also here the markers are detected. The corresponding marker pairs (one out of the patient scan and one out of the impression digitization) are now used to fuse both datasets together. In this way detailed teeth and surrounding soft tissue information is fused with the patient scan, without distorting the facial soft tissues.

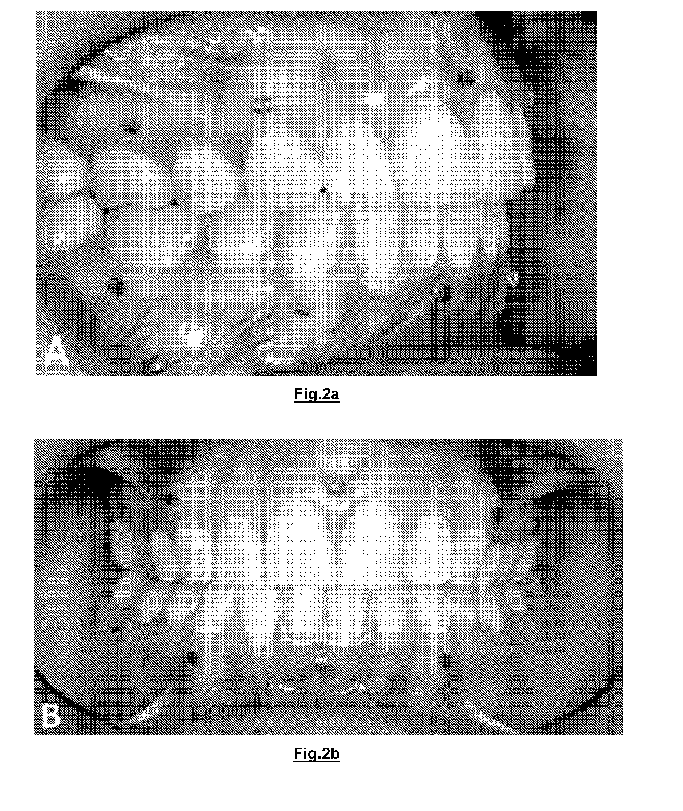

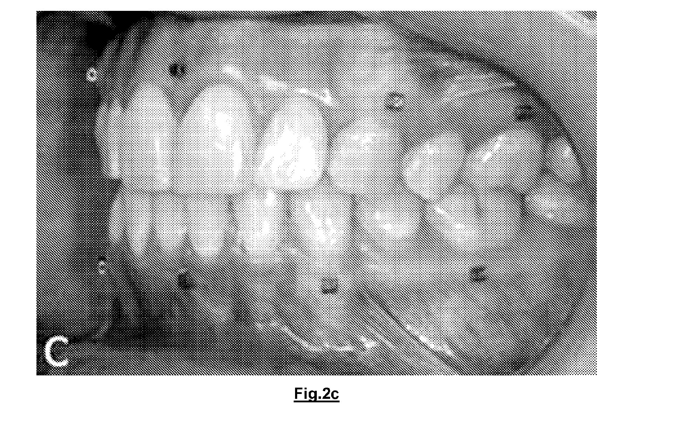

In case the patient is scanned with a CT-scanner, markers can be produced out of titanium. The markers can be glued with tissue adhesive glue to the gingiva. Three or more markers are placed in a random pattern (see Fig.2).

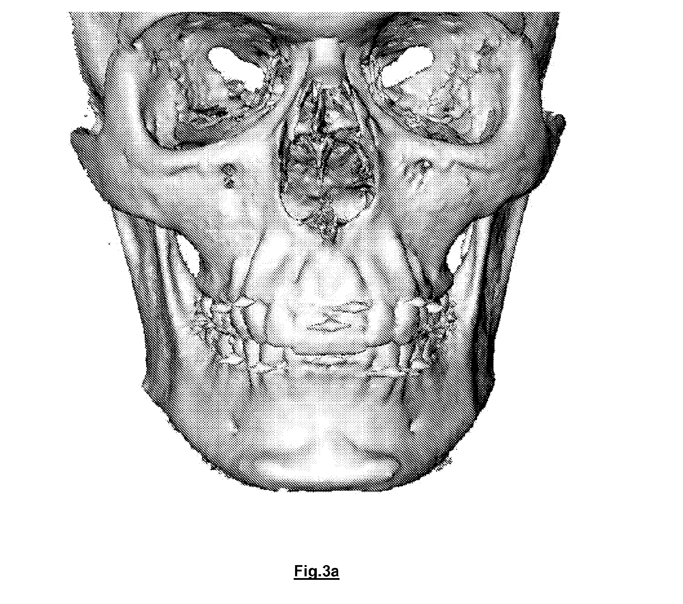

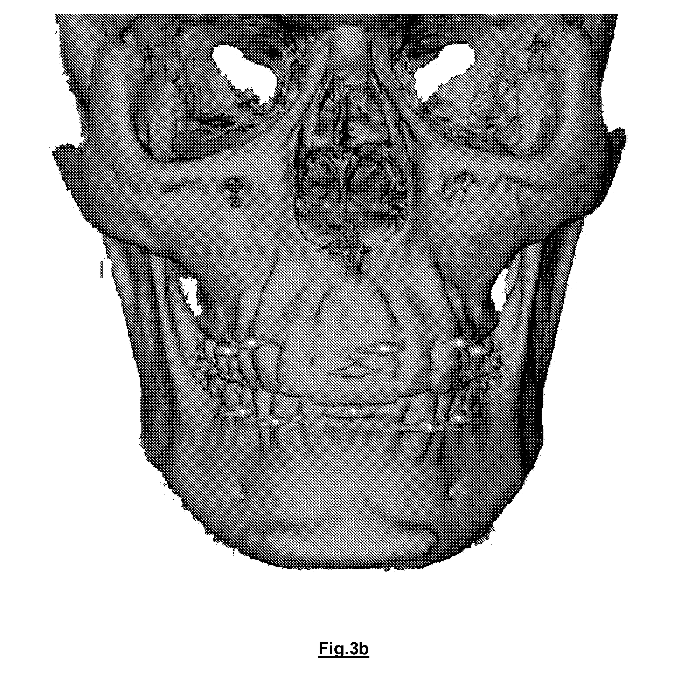

Next the patient is scanned with the CT. The marker can be detected in the CT scan (see Fig.3).

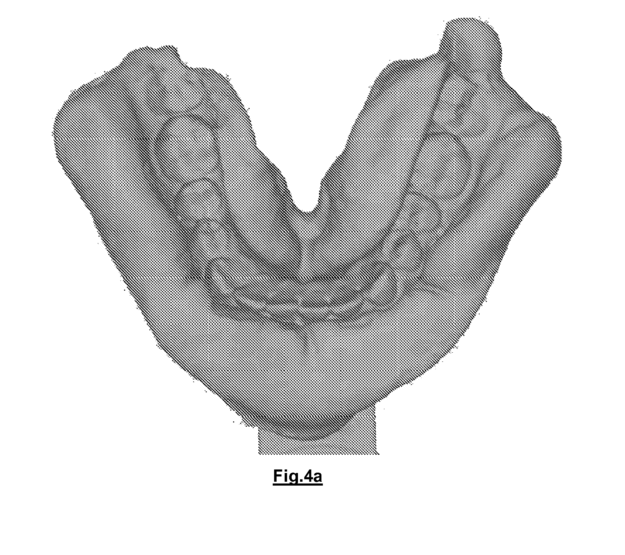

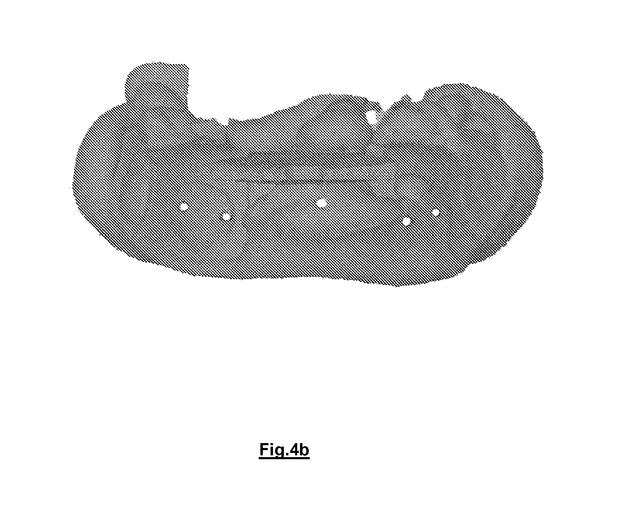

After the scan, impressions are taken. By doing so, the markers are transferred into the impression. Then, the impressions are digitized (see Fig.4)

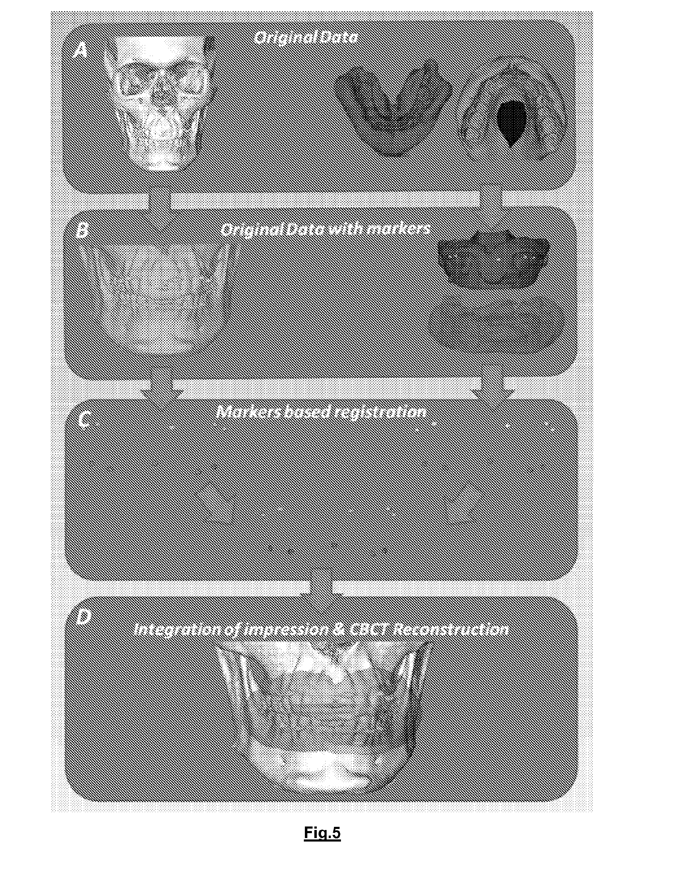

Finally, the digitized impression is fused to the patient based on the markers. The workflow for this possible implementation of the workflow is depicted in Fig.5.

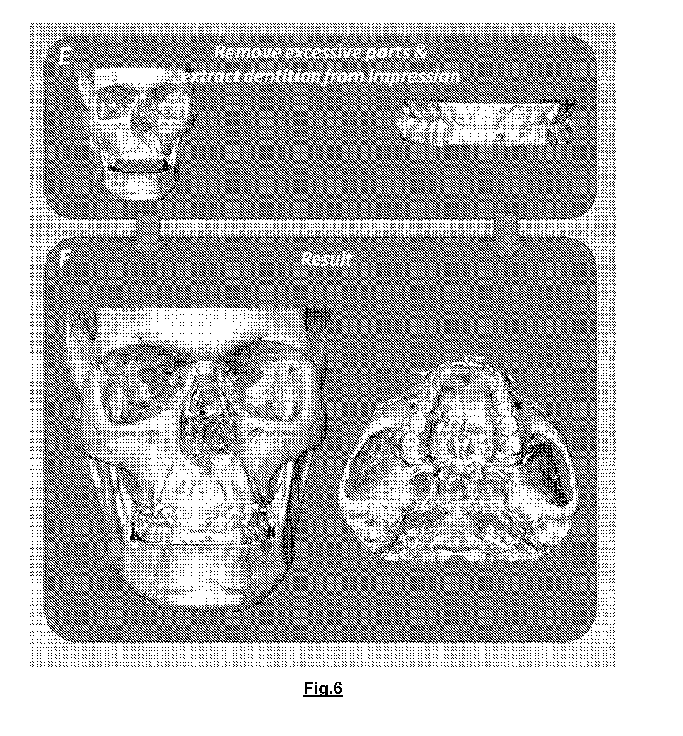

In case that accuracy of the teeth shapes out of the volumetric data set is insufficient, a visualization with the teeth shape retrieved from the impression might be preferred. Therefore,, as an option, the teeth shape out of the patient scan can be replaced with the teeth shape out of the digitized impression. Therefore some access material of the digital impression can be removed, as shown in Fig.6.

As of further option, color information, originating from intra-oral pictures for example, can be added to the digital impression. In this way, the distinction between teeth and surrounding soft tissue becomes clear. Also, the visualization becomes more realistic.

With this invention, detailed teeth shape information and surrounding soft tissue information is fused in the patient scan without deforming the surrounding soft tissues. This method can be executed in a short time and requires minimal additional skills of the clinician.

Although the present invention has been illustrated by reference to specific embodiments, it will be apparent to those skilled in the art that the invention is not limited to the details of the foregoing illustrative embodiments, and that the present invention may be embodied with various changes and modifications without departing from the scope thereof. The present embodiments are therefore to be considered in all respects as illustrative and not restrictive, the scope of the invention being indicated by the appended claims rather than by the foregoing description, and all changes which come within the meaning and range of equivalency of the claims are therefore intended to be embraced therein. In other words, it is contemplated to cover any and all modifications, variations or equivalents that fall within the spirit and scope of the basic underlying principles and whose essential attributes are claimed in this patent application. It will furthermore be understood by the reader of this patent application that the words "comprising" or "comprise" do not exclude other elements or steps, that the words "a" or "an" do not exclude a plurality, and that a single element, such as a computer system, a processor, or another integrated unit may fulfil the functions of several means recited in the claims. Any reference signs in the claims shall not be construed as limiting the respective claims concerned. The terms "first", "second", third", "a", "b", "c", and the like, when used in the description or in the claims are introduced to distinguish between similar elements or steps and are not necessarily describing a sequential or chronological order. Similarly, the terms "top", "bottom", "over", "under", and the like are introduced for descriptive purposes and not necessarily to denote relative positions. It is to be understood that the terms so used are interchangeable under appropriate circumstances and embodiments of the invention are capable of operating according to the present invention in other sequences, or in orientations different from the one(s) described or illustrated above.

## Claims

1. A method for deriving shape information of a person's dentition, the method comprising the steps of
- obtaining a first data set from a three-dimensional scan of said person's dentition with markers fixed in said person's intra-oral cavity,
- digitizing an impression of said person's dentition with said markers fixed, yielding a second data set,
- fusing said first and said second data set based on corresponding pairs of said markers,
- deriving said shape information from said fused first and second data sets.

2. Method for deriving shape information as in claim 1, whereby said markers are fixed to soft tissue in the intra-oral cavity.

3. Method for deriving shape information as in claim 1, whereby said markers are fixed to the teeth or a restoration of the teeth.

4. Method for deriving shape information as in any of claims 1 to 3, wherein said step of digitizing said impression is performed with a volumetric imaging technique or with a surface scanning technique.

5. Method for deriving shape information as in any of the previous claims, wherein said markers are made of titanium.

6. Method for deriving shape information as in any of the previous claims, wherein at least three markers are used.

7. Method for deriving shape information as in claim 6, whereby said at least three markers are placed in a random pattern.

8. Method for deriving shape information as in any of the previous claims, further comprising a step of adding colour information to said impression.

9. Method for deriving shape information as in any of the previous claims, further comprising a step of extracting the dentition from the digitized impression.

10. Method for deriving shape information as in claim 9, comprising a further step wherein said extracted dentition replaces the dentition in the first data set.

11. Method for deriving shape information as in any of the previous claims, further comprising an initial step of acquiring said three-dimensional scan of said person's dentition with markers fixed in said person's intra-oral cavity.

12. Method for deriving shape information as in claim 10, wherein said step of acquiring is followed by a step of making said impression of the patient's dentition with said markers fixed.

13. A program, executable on a programmable device containing instructions, which when executed, perform the method as in any of the claims 1 to 10.
